# EUROPÄISCHE PATENTSCHRIFT

(11) **EP 1 937 155 B1**
(45) Veröffentlichungstag und Bekanntmachung des Hinweises auf die Patenterteilung: **26.09.2018**
(21) Anmeldenummer: 06806368.4
(22) Anmeldetag: 18.10.2006
(51) Int. Cl.: A61B 17/02, A61F 2/46

(54) **BÄNDERSPANN-VORRICHTUNG**
LIGAMENT TENSIONING DEVICE
DISPOSITIF DE TENSION DE LIGAMENTS

(30) Priorität: 18.10.2005 DE 102005049851
(43) Veröffentlichungstag der Anmeldung: 02.07.2008
(73) Patentinhaber: Smith & Nephew Orthopaedics AG, 6343 Rotkreuz (CH)
(72) Erfinder: HAURI, Thomas, CH-5053 Staffelbach (CH); STIFTER, Jan, CH-5424 Unterehrendingen (CH)
(74) Vertreter: Popp, Eugen
(86) Internationale Anmeldenummer: PCT/EP2006/010052
(87) Internationale Veröffentlichungsnummer: WO 2007/045460

(56) Entgegenhaltungen:
- WO-A-2004/078047
- DE-U1- 20 320 501
- US-A- 6 056 756
- US-A1- 2005 020 941
- US-A1- 2005 177 170

## Beschreibung

Die Erfindung betrifft ein Bänderspanngerät zum Einsatz bei der Implantation von Gelenk- oder Skelettimplantaten nach dem Oberbegriff des Anspruchs 1.

Bänderspannvorrichtungen mit dieser Funktion sind bekannt. So ist etwa aus der WO 00/78225 A1 eine Bänderspannvorrichtung bekannt, die neben einem prismatischen, zylindrischen oder plattenförmigen Grundkörper, der eine Anlagefläche zur Anlage an einen ersten, an ein nicht-kugeliges Gelenk angrenzenden Knochen und einen rechten und linken Spannhebel mit zweiten Auflageflächen hat, die auf die gelenkseitige Oberfläche eines zweiten an das Gelenk angrenzenden Knochens zu bringen sind, indem zugeordnete Handgriffe und Bedienungshebel koordiniert betätigt werden. Die einander gegenüberliegenden Teile (Anlageflächen) sind gegeneinander jeweils durch Viergelenk-Hebelgetriebe abgestützt.

Aus der DE 103 48 585 A1 ist eine weitere Bänderspannvorrichtung bekannt, welche eine erste, distale Anlageplatte zur Anlage an ein erstes Skelettteil und eine zweite, proximale Anlageplatte zur Anlage an ein zweites Skelettteil umfasst, wobei die beiden Anlageplatten relativ zueinander durch einen hydraulischen Antrieb verschiebbar und insbesondere über eine mittig verlaufende Achse gegeneinander kippbar sind. Sie sind zudem über einen Grundkörper miteinander verbunden, wobei die distale Anlageplatte insbesondere fest mit diesem verbunden ist.

US 2005/02041 A1 beschreibt einen dynamischen Abstandshalter für die Messung eines Beugungs-Streckungs-Abstandes während einer Knie-Arthoplastie.

US 2005/177170 A1 offenbart eine Vorrichtung für einen chirurgischen Eingriff an einem Knie, wobei die Vorrichtung ein Schienbeinteil, ein feststehendes Oberschenkelteil und ein einstellbares Oberschenkelteil umfasst.

US 6 056 756 A beschreibt ein Instrument zur prothetischen Gelenkersatzchirurgie mit einer Hebevorrichtung zum Stützen eines Knochens und einer Aufspannvorrichtung, um die Vorbereitung des Knochens zum Befestigen einer prothetischen Endkomponente zu erleichtern.

Aus der WO 2004/078047 A1 ist eine weitere, gattungsgemäße Bänderspannvorrichtung bekannt, die eine distale Anlageplatte und zwei dieser gegenüber unabhängig geführte und verschiebliche proximale Anlageplatten umfasst. Die Führung erfolgt hier jeweils über eine doppelte Scharnierverbindung und der Antrieb bevorzugt ebenfalls über eine Hydraulik.

Die bekannten Bänderspannvorrichtungen weisen im Gebrauch nach den jetzt vorliegenden Erfahrungen noch bestimmte Nachteile auf. Diese betreffen insbesondere die erwünschte exakte Ausrichtung zwischen den einander gegenüberliegenden Auflageplatten und den mit einer hydraulischen Antriebsvorrichtung verbundenen Aufwand. Zudem muss das jeweilige Gelenk bei Einsatz der bekannten Bänderspanngeräte offen gehalten werden, da wesentliche Teile der Vorrichtung herausragen.

Der Erfindung liegt daher die Aufgabe zugrunde, ein unaufwendiges und den Anforderungen der Praxis in zuverlässiger Weise genügendes, verbessertes Bänderspanngerät mit vielseitigen Einsatzmöglichkeiten bereitzustellen.

Diese Aufgabe wird durch ein Bänderspanngerät mit den Merkmalen des Anspruchs 1 gelöst. Zweckmäßige Fortbildungen des Erfindungsgedankens sind Gegenstand der abhängigen Ansprüche.

Die Erfindung schließt den wesentlichen Gedanken ein, in das jeweilige Gelenk vollständig einfügbares Bänderspanngerät bereitzustellen, welches es ermöglicht, die Gelenkkapsel nach Einsetzen wieder zu schließen. Hierzu sind insbesondere die Anlageplatten in Anpassung an das jeweilige Gelenk - etwa ein Kniegelenk - zu dimensionieren, und überstehende Teile (wie etwa beim Stand der Technik hervorstehende Handgriffe bzw. Hebel oder Hydraulikleitungen) sind weitestgehend zu vermeiden. Insofern schließt die Erfindung des Gedanken des Vorsehens eines rein internen Antriebes bzw. interner Verschiebemittel zur Relativverschiebung der einander gegenüberliegenden Anlageplatten ein.

Die Erfindung schließt den Gedanken ein, eine in ventral-dorsaler wie auch medial-lateraler Richtung präzise Parallelführung zwischen distaler Anlageplatte und den proximalen Anlageplatten bereitzustellen. Dies wird durch eine geeignete Führung nach dem Scheren-Prinzip realisiert. Nur beispielhaft, nicht erfindungsgemäß, kommen aber auch andere Zwei-Ebenen-Parallelführungen, z.B. unter Verwendung zweier nicht achsenparallel zueinander (insbesondere einen Winkel von 90° zueinander einschließender) Scharniergelenke.

Weiterhin schließt die Erfindung, in einer relativ selbstständigen Ausprägung, den Gedanken ein, als Antrieb zum Voneinander-Abstoßen zwischen der distalen Anlageplatte und den proximalen Anlageplatten jeweils ein separates Antriebselement vorzusehen, und zwar ein ohne Hilfsenergie auskommendes Antriebselement. In dieser Ausprägung gehört zur Erfindung schließlich der Gedanke, jeder proximalen Anlageplatte ein Federelement zuzuordnen, welches in einem Ausgangszustand, in dem die proximale Anlageplatte minimalen Abstand zur distalen Anlageplatte hat, die Antriebsenergie gespeichert hält und sie beim In-Funktion-Setzen des Bänderspanners zur Abstandvergrößerung freigibt. Schließlich gehört zur Erfindung, in bevorzugter Ausführung noch der Gedanke, diesem Federelement bzw. den gegenüberliegenden Anlageplatten eine Verriegelung zur lösbaren Fixierung in der Ausgangslage zuzuordnen.

Grundsätzlich kommt aber auch ein Antrieb mit Hilfsenergie, etwa ein elektromotorischer, elektromagnetischer, hydraulischer oder pneumatischer Antrieb, in Betracht. Dieser kann beide proximale Anlageplatten gemeinsam antreiben, wobei deren Endlage sich im Kräftegleichgewicht mit der umgebenden Kapsel- /Bänderstruktur unterschiedlich einstellen kann. Die genannten Antriebsmittel können aber auch separat für jede proximale Anlageplatte vorgesehen sein.

An dieser Stelle ist darauf hinzuweisen, dass in der vorliegenden Beschreibung und den anhängenden Ansprüchen die Termini "distale Anlageplatte" und "proximale Anlageplatte" unter Bezugnahme auf bevorzugte Gebrauchslagen des Bänderspanners, etwa beim Einsatz im Knie, benutzt werden. Für diesen Einsatzfall könnte man synonym auch von einer Tibia-Anlageplatte bzw. Femur-Anlageplatte sprechen; es sind jedoch ausdrücklich auch Vertauschungen der Anlageplatten im Hinblick auf ihre entferntere oder nähere Lage zum Körperzentrum, (distal bzw. proximal) als zur Erfindung gehörig zu verstehen.

In einer aus derseitiger Sicht bevorzugten Ausführung weist das Federelement ein Druckfederelement auf, das in einem Überdeckungsbereich der proximalen und distalen Anlageplatten angeordnet ist und sich gegen beide abstützt. Hierbei weist insbesondere das Federelement eine Stahl- oder Titan-Schraubenfeder mit geeigneter Federcharakteristik auf. Es zeichnet sich dabei aus durch eine Federsteifigkeit auf die auf, eine vorbestimmte Spannkraft abgestimmt ist, um insbesondere eine über seinen Hub im Wesentlichen konstante Spannkraft im Bereich zwischen 50 und 90 N, insbesondere von 70 N, bereitzustellen.

Im Hinblick auf die besonderen Anforderungen der Realisierung einer relativ hohen und konstanten Spannkraft einerseits und der Realisierung einer möglichst geringen Anfangs-Höhe des Federelementes im gespannten Zustand ist der Einsatz von hierauf zugeschnittenen Sonderbauformen der Druckfeder bevorzugt. Eine aus derzeitiger Sicht bevorzugte Bauform zeichnet sich dadurch aus, dass die Stahl- oder Titan-Schraubenfeder zur Vergrößerung des Hubes konisch oder doppelt-konisch ausgeführt ist und insbesondere eine zum lokalen Windungsdurchmesser indirekt proportionale Steigung aufweist. Der konische Verlauf des Federelementes ist dabei so gewählt, dass die Bauhöhe im gespannten Zustand sich gegenüber einer vergleichbaren Schraubenfeder mit zylindrischer Grundform wesentlich reduziert.

In einer alternativen Ausführung weist das Federelement ein Biegefederelement auf, und zwar beispielsweise eine Stahl-Spiral- oder -Blattfeder.

Die als Parallelführung vorliegende Scherenführung weist bevorzugt zwei nahe gegenüberliegenden Enden der proximalen Anlageplatte angeordnete, also möglichst weit voneinander beabstandete Scherengelenke auf. In verschiedenen Ausführungen der Erfindung können diese in ventral-dorsaler Richtung (hintereinander) oder in medial-lateraler Richtung (nebeneinander) angeordnet sein. Eine vorteilhafte Ausführung der Scherenführung zeichnet sich dadurch aus, dass die Enden der Scherengelenke in Nuten der distalen bzw. proximalen Anlageplatte eingeschoben sind und jeweils ein Schenkel oder jeweils ein Ende beider Schenkel hierin durch senkrecht zur Nut-Erstreckungsrichtung verlaufende Lagerstifte drehfähig fixiert ist.

Eine zusätzlich verbesserte Führung und gegenseitige Fixierung der gegenüberliegenden Anlageplatten wird erreicht, indem an beiden Enden des freien Schenkels oder an dem jeweils freien Ende beider Schenkel ein Gleitstift zur Führung des jeweiligen Schenkels bzw. Endes in einer in Anpassung an den Gleitstift ausgearbeiteten Nut vorgesehen ist. Zur Erhöhung der Steifigkeit und einer noch weiter verbesserten Führung ist bevozugt auch vorgesehen, dass die Scherengelenke über mindestens einen am Ende jeweils einen Schenkels angebrachten Verbindungsstab miteinander verbunden sind.

Zur Anpassung an die konkreten anatomischen Gegebenheiten verschiedener Patienten ist weiterhin vorgesehen, dass die erste und zweite proximale Anlageplatte jeweils ein Ober- und Unterteil umfassen, wobei im Unterteil Mittel zur Halterung des Federelements und der Scherenführung vorgesehen sind und das Oberteil zur Vergrößerung der Dicke der jeweiligen Anlageplatte lösbar auf dem Unterteil fixiert ist. Das Bänderspanngerät kann hierbei mit einem Satz verschieden dicker Oberteile zur besonders präzisen Lösung der erwähnten Anpassungs-Aufgabe zum Einsatz kommen.

Die oben erwähnte Verriegelung wird in zweckmäßiger Weise dadurch gelöst, dass an der distalen Anlageplatte oder der ersten und zweiten proximalen Anlageplatte lösbare Verriegelungsmittel zur unabhängigen Verriegelung der ersten und zweiten proximalen Anlageplatte mit der distalen Anlageplatte in einem minimalen Abstand und mit einer maximalen Federspannung dieser gegenüber vorgesehen sind.

Eine bevorzugte Ausgestaltung ergibt sich dadurch, dass die Verriegelungsmittel jeweils eine an der jeweiligen Anlageplatte schwenkbar gelagerten und in die gegenüberliegende Anlageplatte eingreifenden Haken aufweisen, an dem ein erster Werkzeug-Eingriffsabschnitt zur Betätigung vorgesehen ist. Im Hinblick auf das auf dem Einsatzgebiet des vorgeschlagenen Bänderspanners verfügbare Werkzeug-Reservoir ist der Werkzeug-Eingriffsabschnitt bevorzugt zum Eingriff eines Inbus-Werkzeugs, etwa einem 3,5 mm-Inbus, ausgeführt.

In einer ersten Ausführung der Verriegelungsmittel mit schwenkbaren Haken ist vorgesehen, dass aus dem Material der ersten und zweiten proximalen Anlageplatte in räumlicher Zuordnung zum an der distalen Anlageplatte angelenkten Haken ein Eingriffsabschnitt für diesen ausgearbeitet ist.

Eine zweite Ausführung sieht vor, dass jeweils ein erster Lagerstift eines Scherengelenks der ersten und zweiten proximalen Anlageplatte über deren Außenrand vorsteht und relativ zum an der distalen Anlageplatte schwenkbaren Haken so angeordnet ist, dass er das Widerlager der Verriegelungsmittel bildet. Hierbei - oder auch unabhängig davon - sieht eine sinnreiche konstruktive Lösung des weiteren vor, dass jeweils ein zweiter Lagerstift eines Scherengelenks der ersten und zweiten proximalen Anlageplatte über deren Außenrand vorsteht und relativ zum an der distalen Anlageplatte schwenkbaren Haken so angeordnet ist, dass er dessen Schwenkachse bildet.

Da wegen der hohen erforderlichen Kräfte auch zur Positionierung des Bänderspanners am Einsatzort zweckmäßigerweise ein Werkzeug eingesetzt wird, ist bevorzugt an den Seitenkanten der distalen Anlageplatte und der ersten und zweiten proximalen Anlageplatte jeweils ein zweiter Werkzeug-Eingriffsabschnitt, insbesondere eine zylindrische Ausnehmung, zum Eingriff eines Positionierungswerkzeugs vorgesehen. Das Vorsehen eines separaten Werkzeuges ist im Hinblick auf die Realisierung eines vollständig gelenkintegrierbaren Bänderspanners von besonderem Vorteil.

Eine weitere zweckmäßige Ausführung sieht eine Ausbildung zum lösbaren Ankoppeln eines Sizers vor, welcher das proximale und distale Skelettteil in Beugung derart koppelt, dass eine Achsenübertragung von dem einen Skelettteil auf das andere unter Berücksichtigung der Bänderspannung möglich ist. Grundsätzlich umfasst das Ankoppeln auch ein einfaches Anlegen an geeignet bereitgestellte Anlageflächen, bevorzugt jedoch Verbindungsmittel zur Fixierung des Sizers an der distalen Anlageplatte. In einer aus derzeitiger Sicht zweckmäßigen Ausführung umfassen die Verbindungsmittel zwei klammerartige Fortsätze an der distalen Anlageplatte, welche jeweils eine Seitenkante der proximalen Anlageplatten umgreifend zu einer Grundplatte des Sizers geführt sind.

Als zum Schutzbereich der Erfindung gehörig sollen auch Anordnungen aus dem vorgeschlagenen Bänderspanngerät und einem angepassten Positionierungswerkzeug und/oder einem daran angepassten Sizer angesehen werden.

Vorteile und Zweckmäßigkeiten der Erfindung ergeben sich im übrigen aus den abhängigen Ansprüchen sowie der nachfolgenden Beschreibung bevorzugter Ausführungsbeispiele anhand der Figuren. Von diesen zeigen:
- Fig. 1: eine perspektivische Ansicht eines erfindungsgemäßen Bänderspanngerätes ventral schräg von oben, teilweise als Explosionsdarstellung, mit angesetztem Positionierungswerkzeug,
- Fig. 2: eine perspektivische Ansicht des Bänderspanngerätes nach Fig. 1 ventral schräg von oben, mit abgenommener zweiter proximaler Anlageplatte und den zugehörigen Verschiebemitteln im voll ausgefahrenen Zustand,
- Fig. 3: eine perspektivische Ansicht des Bänderspanngerätes nach Fig. 1 und 2, mit abgenommener zweiter proximaler Anlageplatte und Verschiebemitteln in Zwischenstellung, dorsal schräg von oben,
- Fig. 4: eine perspektivische Ansicht des Bänderspanngerätes nach Fig. 1 - 3, mit abgenommener zweiter proximaler Anlageplatte, ventral schräg von unten,
- Fig. 5: eine perspektivische Darstellung einer als Federelement beim Bänderspanngerät nach Fig. 1 - 4 einsetzbaren Schraubenfeder in doppelt-konischer Ausführung,
- Fig. 6A - 6D: verschiedene Ansichten des Bänderspanngerätes nach Fig. 1 in seinem in ein Kniegelenk eingesetzten Gebrauchszustand, und
- Fig. 7A - 7D: verschiedene Ansichten des Bänderspanngerätes nach Fig. 1 in seinem in ein Kniegelenk eingesetzten Gebrauchszustand, mit einem angekoppelten Sizer.

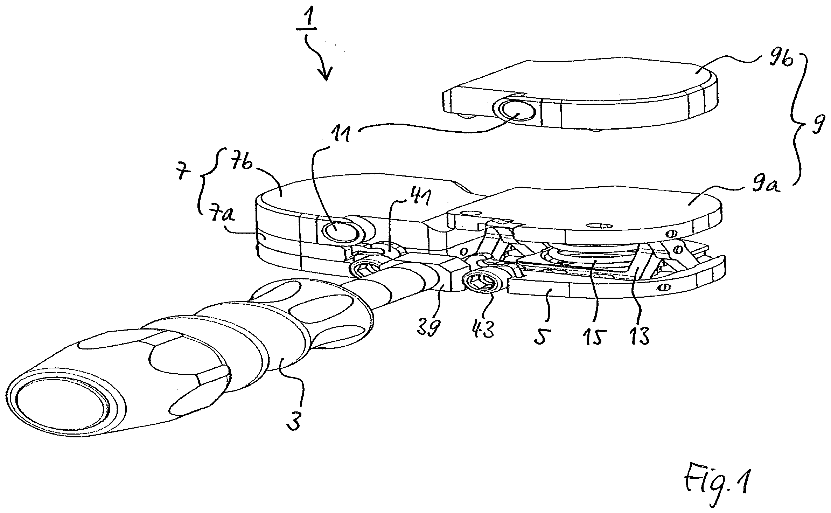

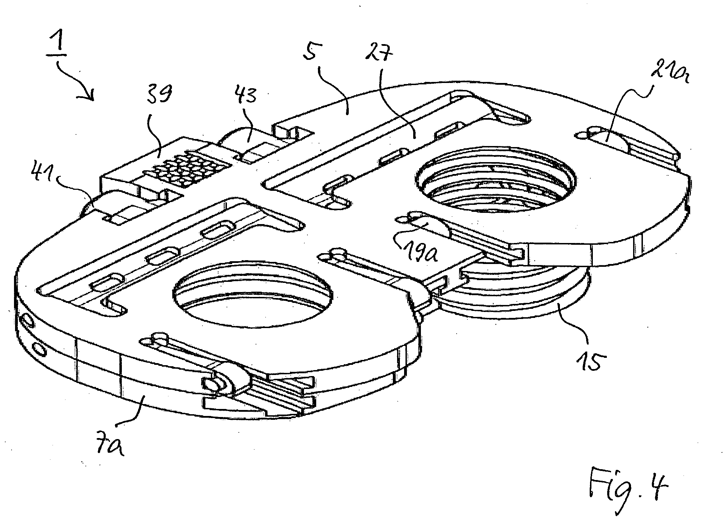

Fig. 1 - 4 zeigen in verschiedenen Ansichten und verschiedenen Zuständen ein Bänderspanngerät 1, wobei Fig. 1 dieses im Zusammenwirken mit einem Positionierungswerkzeug darstellt.

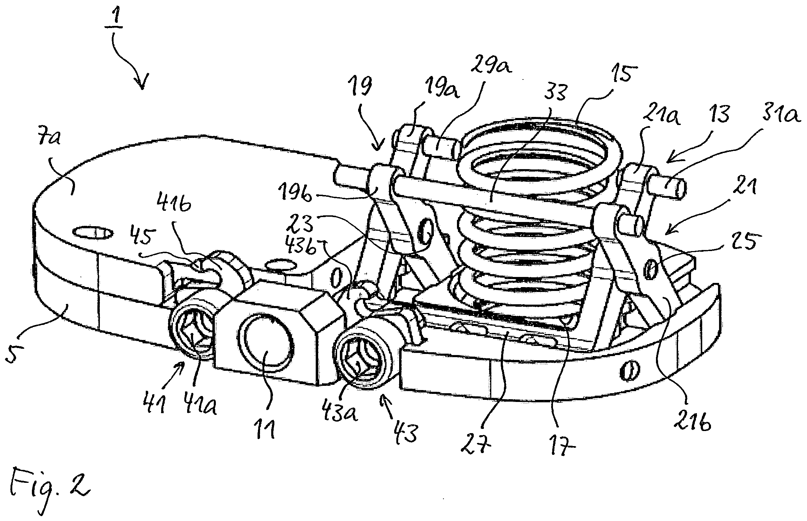

Das Bänderspanngerät 1 umfasst eine in der Draufsicht annähernd nierenförmige distale Anlageplatte 5 und zwei dieser parallel gegenüberliegende und sie jeweils annähernd hälftig überdeckende proximale Anlageplatten 7 und 9, die jeweils ein Unterteil 7a, 9a und ein Oberteil 7b, 9b umfassen. Zur Positionierung des Bänderspanngerätes 1 mit Hilfe des Positionierungswerkzeuges 3 sind an den frontalen Seitenkanten der distalen und proximalen Anlageplatten jeweils zylindrische Ausnehmungen 11 als Eingriffsabschnitte vorgesehen. (In Fig. 1 sind hiervon die Ausnehmungen in den proximalen Anlageplatten, genauer: deren Oberteilen 7b, 9b, gezeigt, während Fig. 2 die zentrale Ausnehmung 11 in der distalen Anlageplatte 5 zeigt.)

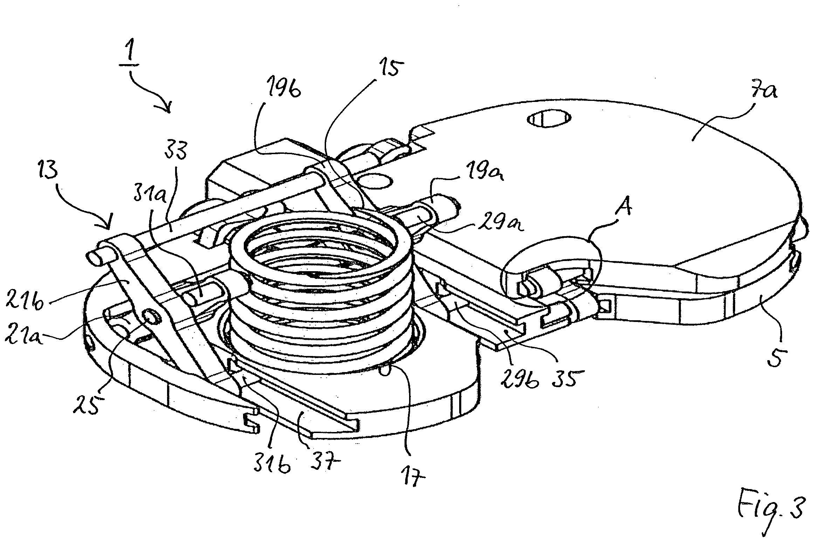

Die proximalen Anlageplatten 7 und 9 (oder, genauer: deren Unterteile 7a, 9a) sind jeweils über eine Scherenführung 13 miteinander verbunden und durch eine Stahl-Druckfeder 15 als Federelement gegeneinander vorgespannt. Wie am besten in Fig. 2 und 3 zu erkennen ist, sitzt die Schraubenfeder 15 in einer angepasst kreisförmigen Ausnehmung 17 auf der Oberseite der distalen Anlageplatte 5; eine ähnliche (nicht dargestellte) Lagerung ist an der Unterseite der jeweils zugehörenden proximalen Anlageplatte vorgesehen.

Die Scherenführung 13 umfasst ein mediales und laterales Scherengelenk 19 bzw. 21, die jeweils zwei über eine Drehachse 23 bzw. 25 drehbar verbundene Schenkel 19a, 19b bzw. 21a, 21b umfassen. Die Schenkel 19a, 21a sind über eine einstückig angeformte Brücke bzw. Verbindungsstange 27 an jeweils einem Ende miteinander verbunden, während in ihr anderes Ende jeweils ein Gleitstift bzw. -zapfen 29a bzw. 31a zur Verbindung mit dem (in Fig. 2 und 3 fortgelassenen) Unterteil 9a der zweiten Anlageplatte eingesetzt ist. In ähnlicher Weise ist jeweils ein Ende der Schenkel 19b, 21b über einen senkrecht zur Erstreckungsebene eingefügten Verbindungsstab 33 verbunden, der über die mediale bzw. laterale Außenseite des jeweiligen Schenkels hinausragt und dort weitere (nicht gesondert bezeichnete) Gleitstifte ausbildet.

An den gegenüberliegenden Enden der Schenkel 19b, 21b ist jeweils wiederum ein separater Gleitstift 29b bzw. 31b vorgesehen. Diese Gleitstifte 29b, 31b greifen (wie am besten in Fig. 3 zu erkennen ist) in entsprechend bemessener, in Gebrauchslage des Bänderspanngerätes dorsal-ventral verlaufende Nuten 35, 37 der distalen Anlageplatte 5 ein und fixieren die Scherenführung hiermit gleitbar in der distalen Anlageplatte 5. (Wie in dem das Unterteil 7a der ersten proximalen Anlageplatte 7 darstellenden Abschnitt von Fig. 3 ansatzweise zu erkennen ist - siehe Detail "A"-, ist in den proximalen Anlageplatten ein entsprechender, hier nicht gesondert bezifferter Nutaufbau vorgesehen.)

Wie am besten in Fig. 2 zu erkennen, sind an der ventralen Seitenkante (Vorderkante) der distalen Anlageplatte 5 zu beiden Seiten eines die zylindrischen Ausnehmung 11 enthaltenden Fortsatzes zwei schwenkbare Hakenelemente 41, 43 angebracht, die jeweils einen Inbus-Eingriffsabschnitt 41a bzw. 43a und einen angeformten Hakenabschnitt 41b bzw. 43b umfassen. Die Hakenabschnitte greifen, wie in Fig. 2 im Bereich der ersten proximalen Anlageplatte zu erkennen ist, in einer zur Hakengestalt korrespondierend geformten Ausnehmung an der Oberseite des jeweiligen Anlageplatten-Unterteils ein - wobei in Fig. 2 nur die Ausnehmung 45 im Unterteil 7a der ersten proximalen Anlageplatte dargestellt ist. Vermittels dieses Eingriffes werden die proximalen Anlageplatten in minimalen Abstand zur distalen Anlageplatte gehalten. Durch Drehen des jeweiligen Hakenelementes 41 bzw. 43 mit einem passenden Werkzeug wird diese Verriegelung gelöst, und die jeweilige proximale Anlageplatte kann sich unter der Vorspannung der zugehörigen Druckfeder 15 von der distalen Anlageplatte wegbewegen, bis ihre Bewegung im Kräftegleichgewicht mit der am Einsatzort wirkenden Kapsel-/Bänderspannung zum Stillstand kommt.

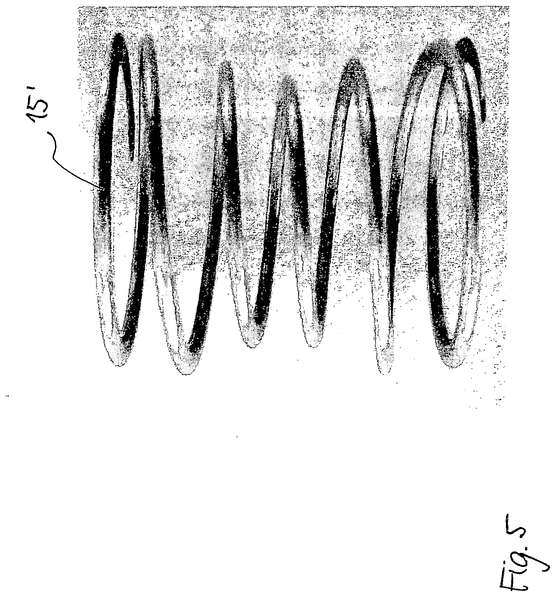

Während in den Figuren 1 - 4 als Federelement eine zylindrische Schraubenfeder 15 gezeigt ist, zeigt Fig. 5 als Abwandlung eine doppelt-konische Schraubenfeder 15', die aufgrund ihrer im gespannten Zustand geringeren Bauhöhe beim Bänderspanngerät 1 nach Fig. 1 - 4 bevorzugt eingesetzt werden kann. Hierbei kann (in nicht dargestellter Ausführung) die Steigung der Wicklung im unteren und oberen Bereich größeren Durchmessers geringer sein als im mittleren Bereich, damit eine einwandfreie Federwirkung über den gesamten Hub gewährleistet ist.

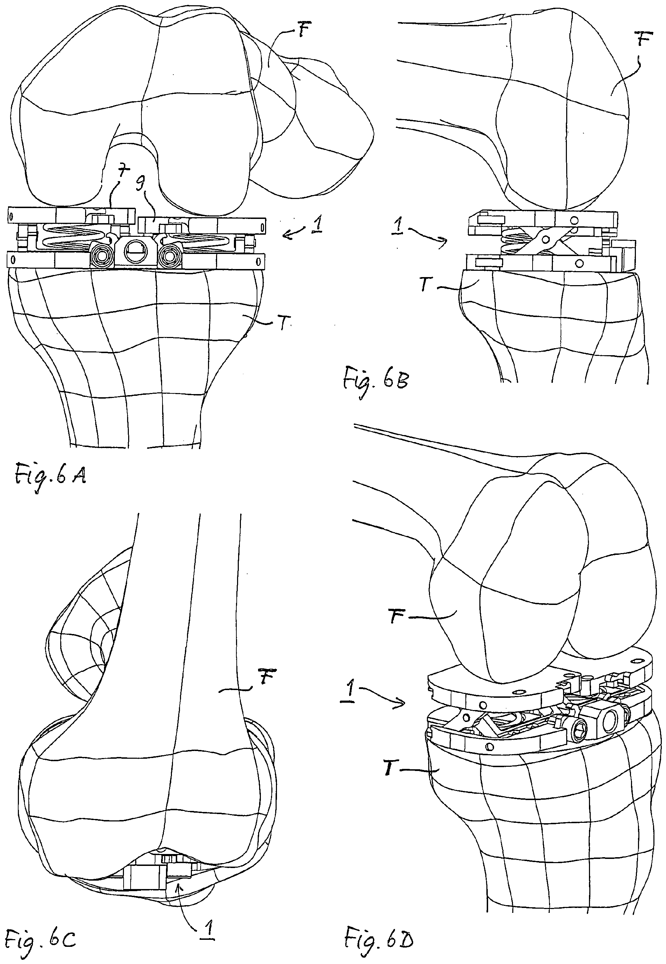

Da oben beschriebene Bänderspanngerät 1 ist zum Einsatz in einem Kniegelenk ausgebildet, und in Fig. 6A - 6D ist dieser Gebrauchszustand in verschiedenen Ansichten gezeigt. Gut zu erkennen ist die Anpassung der Dimensionen, insbesondere des Längen/Breiten-Verhältnisses und die Ausführung der beiden proximalen Anlageplatten 7, 9 an die anatomischen Verhältnisse des Kniegelenks und speziell die Gesamtabmessungen und Größenrelationen der Tibia T und des (resezierten) Femurs F.

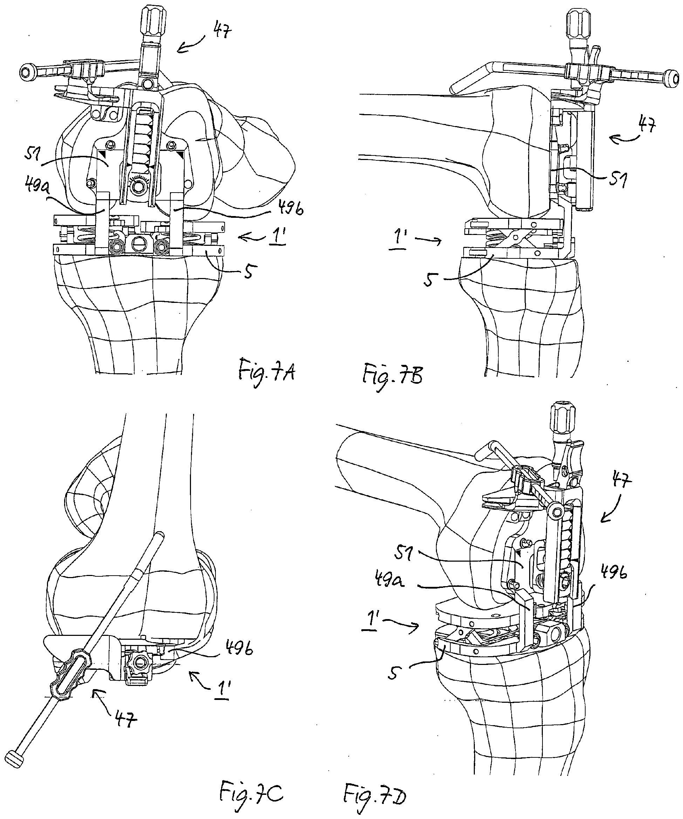

In Fig. 7A - 7D ist, in vergleichbarer Darstellung wie in Fig. 6A - 6D, skizzenartig als modifizierte Ausführung ein Bänderspanngerät 1' mit einem angekoppelten Sizer 47 gezeigt. Ein Sizer dient bekanntermaßen zur intraoperativen Kopplung von Tibia und Femur in Beugung derart, dass eine Achsenübertragung der tibialen Achse unter Berücksichtigung der Bandspannung auf den Femur erfolgen kann. Hiermit kann außerdem die optimale Größe und Lage eines Knie-Implantates in ventral-dorsaler Richtung und in Rotation definiert werden. Die technischen Merkmale des Sizers sind nicht Gegenstand der vorliegenden Erfindung und werden daher hier nicht beschrieben. Hinzuweisen ist jedoch auf das Vorsehen von Verbindungsmitteln zwischen dem Bänderspanngerät 1' und dem Sizer 47, nämlich zwei klammerartigen Fortsätzen bzw. Verbindungsbügeln 49a, 49b an der distalen Anlageplatte 5, welche die (hier nicht bezeichneten) proximalen Anlageplatten an einer Seitenkante der selben übergreifen und eine Verbindung mit einer Grundplatte 51 des Sizers 47 herstellen.

Die Ausführung der Erfindung beschränkt sich nicht auf das hier erläuterte Ausführungsbeispiel und die zuletzt erwähnte Modifikation des Federelementes, sondern ist ebenso in einer Vielzahl von Abwandlungen möglich, die im Rahmen fachgemäßen Handelns liegen. So ist sie insbesondere auch mit einer Scherenführung möglich, die gegenüber der dargestellten Ausführungsform um 90° gedreht angeordnet ist und bei der im übrigen ein Teil der Gleitstifte bzw. -zapfen zugleich als Lagerzapfen zur ortsfesten Fixierung jeweils eines Schenkelendes in der zugehörigen Anlageplatte ausgebildet sein kann. In einer solchen Ausführung ist es zudem möglich, jeweils einen solchen Lagerzapfen zugleich als Schwenkachse der schwenkbaren Verriegelungshaken zu nutzen. Weiterhin können bei den proximalen Anlageplatten die Oberteile auch fortgelassen sein, und zahlreiche Freiheitsgrade bestehen hinsichtlich der exakten Form und Abmessungs-Relationen der Anlageplatten.

### Bezugszeichenliste

- 1: Bänderspanngerät
- 2: Positionierungswerkzeug
- 5: distale Anlageplatte
- 7, 9: proximale Anlageplatten
- 7a, 9a: Unterteile der proximalen Anlageplatten
- 7b, 9b: Oberteile der proximalen Anlageplatten
- 11: zylindrische Ausnehmung
- 13: Scherenführung
- 15: zylindrische Schraubenfeder
- 15': doppelt-konische Schraubenfeder
- 17: kreisförmige Ausnehmung
- 19: mediales Scherengelenk
- 19a, 19b: Schenkel des medialen Scherengelenks
- 21: laterales Scherengelenk
- 21a, 21b: Schenkel des lateralen Scherengelenks
- 23: Drehachse des medialen Scherengelenks
- 25: Drehachse des lateralen Scherengelenks
- 27: Verbindungsstange
- 29a, 29b, 31a, 31b: Gleitstifte
- 35, 37: Nuten
- 41, 43: Hakenelemente
- 41a, 43a: Inbus-Eingriffsabschnitte
- 41b, 43b: angeformte Hakenabschnitte
- 45: Ausnehmung
- 47: Sizer
- 49a, 49b: Fortsätze
- 51: Grundplatte

## Patentansprüche

1. Bänderspanngerät (1) zur Aktivierung des Band- und/oder Kapselapparates bei der Implantation eines Gelenkimplantats, mit einer distalen Anlageplatte (5) zur Anlage an ein distales Skelettteil und einer ersten und zweiten, in einer ersten Gebrauchslage die distale Anlageplatte (5) jeweils partiell überdeckenden proximalen Anlageplatte (7, 9) zur Anlage an ein proximales Skelettteil und Mitteln zur Relativ-Verschiebung der proximalen Anlageplatten (7, 9) zur Vergrößerung ihres Abstandes zur distalen Anlageplatte,
wobei sämtliche wesentlichen Komponenten des Bänderspanngerätes, insbesondere die distale Anlageplatte (5) und erste und zweite proximale Anlageplatte (7, 9), zur Unterbringung zwischen gelenk-benachbarten Abschnitten des distalen und proximalen Skelettteils dimensioniert sind, derart, dass eine Gelenkkapsel nach Einsetzen des Bänderspanngerätes wieder geschlossen werden kann, **dadurch gekennzeichnet, dass** die Verschiebemittel eine die
jeweilige proximale Anlageplatte in Parallel-Lage zur distalen Anlageplatte abstützende Scherenführung (13) aufweisen, welche zwischen der distalen und der jeweiligen proximalen Anlageplatte derart torsionssteif ausgebildet ist, dass sie eine in ventral-dorsaler wie auch in medial-lateraler Richtung präzise Parallelführung zwischen distaler Anlageplatte und proximaler Anlageplatte bereitstellt.

2. Bänderspanngerät (1) nach Anspruch 1,
**gekennzeichnet durch**
ausschließlich interne Mittel zur Relativ-Verschiebung der proximalen Anlageplatten gegenüber der distalen Anlageplatte.

3. Bänderspanngerät (1) nach einem der vorangegangenen Ansprüche
**dadurch gekennzeichnet, dass**
die Verschiebemittel mindestens ein Federelement (15) zum Antrieb der proximalen Anlageplatten aufweisen, welches in einem Ausgangszustand, in dem die proximalen Anlageplatten (7, 9) minimalen Abstand zur distalen Anlageplatte (5) haben, die Antriebsenergie gespeichert hält und sie beim In-Funktion-Setzen des Bänderspanners zur Abstandvergrößerung freigibt.

4. Bänderspanngerät (1) nach Anspruch 3,
**dadurch gekennzeichnet, dass**
die Verschiebemittel in Zuordnung zur ersten und zweiten proximalen Anlageplatte jeweils ein Federelement (15) zum Antrieb der proximalen Anlageplatten aufweisen.

5. Bänderspanngerät (1) nach Anspruch 3 oder 4,
**dadurch gekennzeichnet, dass**
das oder jedes Federelement (15) derart ausgebildet ist, dass es verriegelbar ist.

6. Bänderspanngerät (1) nach einem der Ansprüche 3 bis 5,
**dadurch gekennzeichnet, dass**
das Federelement (15) ein Druckfederelement aufweist, das in einem Überdeckungsbereich der proximalen und distalen Anlageplatten angeordnet ist.

7. Bänderspanngerät (1) nach Anspruch 6,
**dadurch gekennzeichnet, dass**
das Federelement (15) eine Stahl- oder Titan-Schraubenfeder aufweist, die zur Vergrößerung des Hubes konisch oder doppelt-konisch ausgeführt ist und insbesondere eine zum lokalen Windungsdurchmesser indirekt proportionale Steigung aufweist.

8. Bänderspanngerät (1) nach Anspruch 3 oder 4,
**dadurch gekennzeichnet, dass**
das Federelement (15) ein Biegefederelement aufweist.

9. Bänderspanngerät (1) nach Anspruch 8,
**dadurch gekennzeichnet, dass**
das Federelement (15) eine Metall-Spiral- oder -Blattfeder aufweist.

10. Bänderspanngerät (1) nach einem der vorangehenden Ansprüche,
**dadurch gekennzeichnet, dass**
die Scherenführung (13) zwei nahe gegenüberliegenden Enden der proximalen Anlageplatte angeordnete Scherengelenke aufweist.

11. Bänderspanngerät (1) nach Anspruch 10,
**dadurch gekennzeichnet, dass**
die Enden der Scherengelenke in Nuten der distalen bzw. proximalen Anlageplatte eingeschoben sind und jeweils ein Schenkel oder jeweils ein Ende beider Schenkel hierin durch senkrecht zur Nut-Erstreckungsrichtung verlaufende Lager- und Gleitzapfen drehfähig fixiert ist.

12. Bänderspanngerät (1) nach Anspruch 11,
**dadurch gekennzeichnet, dass**
an beiden Enden des freien Schenkels oder an dem jeweils freien Ende beider Schenkel ein Gleitzapfen zur Führung des jeweiligen Schenkels bzw. Endes in einer in Anpassung an den Gleitzapfen ausgearbeiteten Nut der distalen bzw. proximalen Anlageplatte vorgesehen ist.

13. Bänderspanngerät (1) nach einem der Ansprüche 10 bis 12,
**dadurch gekennzeichnet, dass**
die Scherengelenke über einen am Ende jeweils einen Schenkels angebrachten Verbindungsstab miteinander verbunden sind.

14. Bänderspanngerät (1) nach einem der vorangehenden Ansprüche,
**dadurch gekennzeichnet, dass**
die erste und zweite proximale Anlageplatte (7, 9) jeweils ein Ober- und Unterteil (7a, 7b, 9a, 9b) umfassen, wobei im Unterteil (7a, 9a) Mittel zur Halterung der Scherenführung (13) und optional des Federelementes (15) vorgesehen sind und das Oberteil (7b, 9b) zur wahlweisen Vergrößerung der Dicke der jeweiligen Anlageplatte lösbar auf dem Unterteil fixiert ist.

15. Bänderspanngerät (1) nach einem der vorangehenden Ansprüche,
**dadurch gekennzeichnet, dass**
an der distalen Anlageplatte (5) oder der ersten und zweiten proximalen Anlageplatte (7, 9) lösbare Verriegelungsmittel zur unabhängigen Verriegelung der ersten und zweiten proximalen Anlageplatte mit der distalen Anlageplatte in einem minimalen Abstand und dieser gegenüber vorgesehen sind.

16. Bänderspanngerät (1) nach Anspruch 15,
**dadurch gekennzeichnet, dass**
die Verriegelungsmittel jeweils eine an der jeweiligen Anlageplatte schwenkbar gelagerten und in die gegenüberliegende Anlageplatte eingreifenden Haken aufweisen, an dem ein erster Werkzeug-Eingriffsabschnitt zur Betätigung vorgesehen ist.

17. Bänderspanngerät (1) nach Anspruch 16,
**dadurch gekennzeichnet, dass**
der erste Werkzeug-Eingriffsabschnitt zum Eingriff eines Standard-Inbus-Schlüssels ausgeformt ist.

18. Bänderspanngerät (1) nach Anspruch 16 oder 17,
**dadurch gekennzeichnet, dass**
aus dem Material der ersten und zweiten proximalen Anlageplatte (7, 9) in räumlicher Zuordnung zum an der distalen Anlageplatte (5) angelenkten Haken ein Eingriffsabschnitt für diesen ausgearbeitet ist.

19. Bänderspanngerät (1) nach einem der Ansprüche 16 bis 18,
**dadurch gekennzeichnet, dass**
jeweils ein erster Lagerstift eines Scherengelenks der ersten und zweiten proximalen Anlageplatte (7, 9) über deren Außenrand vorsteht und relativ zum an der distalen Anlageplatte (5) schwenkbaren Haken so angeordnet ist, dass er das Widerlager der Verriegelungsmittel bildet.

20. Bänderspanngerät (1) nach einem der Ansprüche 16 bis 19,
**dadurch gekennzeichnet, dass**
jeweils ein zweiter Lagerstift eines Scherengelenks der ersten und zweiten proximalen Anlageplatte (7, 9) über deren Außenrand vorsteht und relativ zum an der distalen Anlageplatte (5) schwenkbaren Haken so angeordnet ist, dass er dessen Schwenkachse bildet.

21. Bänderspanngerät (1) nach einem der vorangehenden Ansprüche,
**dadurch gekennzeichnet, dass**
an den Seitenkanten der distalen Anlageplatte (5) und der ersten und zweiten proximalen Anlageplatte (7, 9) jeweils ein zweiter Werkzeug-Eingriffsabschnitt, insbesondere eine zylindrische Ausnehmung, zum Eingriff eines Positionierungswerkzeugs vorgesehen ist.

22. Bänderspanngerät (1) nach einem der Ansprüche 3 bis 21,
**gekennzeichnet durch**
ein in der Federsteifigkeit auf eine vorbestimmte Spannkraft abgestimmtes Federelement (15), welches insbesondere eine über seinen Hub im Wesentlichen konstante Spannkraft im Bereich zwischen 50 und 90 N, insbesondere von 70 N, bereitstellt.

23. Bänderspanngerät (1) nach einem der vorangehenden Ansprüche,
**dadurch gekennzeichnet, dass**
das Längen/Breiten-Verhältnis, also die Abmessung in medial-lateraler Richtung gegenüber derjenigen in ventral-dorsaler Richtung, an die Proportionen eines geschnittenen gelenknahen Abschnitts des benachbarten Skelettteils angepasst sind.

24. Bänderspanngerät (1) nach einem der vorangehenden Ansprüche,
**gekennzeichnet durch**
die Ausführung in Anpassung an ein Kniegelenk, wobei die benachbarten Skelettteile ein Femur und eine Tibia sind.

25. Bänderspanngerät (1) nach Anspruch 23 und 24,
**dadurch gekennzeichnet, dass**
das Längen/Breiten-Verhältnis entsprechend dem einer proximalen geschnittenen Tibia 3/2 beträgt.

26. Bänderspanngerät (1) nach einem der vorangehenden Ansprüche,
**gekennzeichnet durch**
eine Ausbildung zum lösbaren Ankoppeln eines Sizers, welcher das proximale und distale Skelettteil in Beugung derart koppelt, dass eine Achsenübertragung von dem einen Skelettteil auf das andere unter Berücksichtigung der Bänderspannung möglich ist.

27. Bänderspanngerät (1) nach Anspruch 26,
**gekennzeichnet durch**
Verbindungsmittel zur Fixierung des Sizers an der distalen Anlageplatte (5).

28. Bänderspanngerät (1) nach Anspruch 27,
**dadurch gekennzeichnet, dass**
die Verbindungsmittel zwei klammerartige Fortsätze an der distalen Anlageplatte aufweisen, welche jeweils eine Seitenkante der proximalen Anlageplatten (7, 9) umgreifend zu einer Grundplatte des Sizers geführt sind.

29. Anordnung aus einem Bänderspanngerät (1) nach einem der vorangehenden Ansprüche und einem Sizer, die insbesondere in Anpassung an ein Kniegelenk ausgeführt und miteinander lösbar gekoppelt sind.

## Claims

1. A ligament-tensioning device (1) for activation of the ligament and/or capsule system during the implantation of a joint implant, comprising a distal bearing plate (5) to bear against a distal skeletal part and a first and second proximal bearing plate (7, 9), each of which at least partially overlaps said distal bearing plate (5) in a first usage position, to bear against a proximal skeletal part and means for relative displacement of said proximal bearing plates (7, 9) to increase their spacing from the distal bearing plate,
wherein all the essential components of said ligament-tensioning device, in particular the distal bearing plate (5) and first and second proximal bearing plate (7, 9) are dimensioned such that they can be accommodated between portions of the distal and proximal skeletal parts adjacent to the joint in such a manner that a joint capsule can be closed again after insertion of the ligament-tensioning device,
**characterized in that**
the displacement means comprises scissor-type guide (13) which supports the respective proximal bearing plates in a parallel position with respect to the distal bearing plate, which is configured to be torsionally stiff construction between the distal bearing plate and the respective proximal bearing plate in such a manner that said guide provides precise parallel guidance between the distal bearing plate and the proximal bearing plates in both the ventral-dorsal and the medial-lateral directions.

2. The ligament-tensioning device (1) according to claim 1,
**characterized by**
exclusively internal means for the relative displacement of the proximal bearing plates with respect to the distal bearing plate.

3. The ligament-tensioning device (1) according to one of the preceding claims,
**characterized in that**
said displacement means comprises at least one spring element (15) to drive said proximal bearing plates, which spring element keeps the drive energy stored in a starting state in which said proximal bearing plates (7, 9) have a minimum spacing from said distal bearing plate (5), and releases said energy to increase the spacing when the ligament-tensioning device is actuated.

4. The ligament-tensioning device (1) according to claim 3,
**characterized in that**
the displacement means has, assigned to each of said first and second bearing plates, a spring element (15) for driving the proximal bearing plates.

5. The ligament-tensioning device (1) according to claim 3 or 4,
**characterized in that**
the or each spring element (15) is configured in such a manner that it is lockable.

6. The ligament-tensioning device (1) according to one of claims 3 to 5,
**characterized in that**
the spring element (15) comprises a compression spring element arranged in a region of overlap between said proximal and distal bearing plates.

7. The ligament-tensioning device (1) according to claim 6,
**characterized in that**
the spring element (15) comprises a steel or titanium helical spring which is designed to be conical or double-conical in order to increase the travel and in particular has a pitch indirectly proportional to the local winding diameter.

8. The ligament-tensioning device (1) according to claim 3 or 4,
**characterized in that**
the spring element (15) comprises a flexural spring element.

9. The ligament-tensioning device (1) according to claim 8,
**characterized in that**
said spring element (15) comprises a metal spiral spring or a leaf spring.

10. The ligament-tensioning device (1) according to one of the preceding claims,
**characterized in that**
the
scissor-type guide (13) comprises two scissor joints arranged close to opposite ends of said proximal bearing plate.

11. The ligament-tensioning device (1) according to claim 10,
**characterized in that**
the ends of the scissor joints are inserted in grooves in said distal or proximal bearing plates and one limb or one end of one limb of each joint is rotatably fixed herein by bearing pins or slide pegs extending perpendicular to the direction of extension of the groove.

12. The ligament-tensioning device (1) according to claim 11,
**characterized in that**
at both ends of the free limb or at the respectively free end of both limbs there is provided a slide peg for guiding the respective limb or end in a groove in said distal or proximal bearing plate, which groove is formed to match the slide peg.

13. The ligament-tensioning device (1) according to one of claims 10 to 12,
**characterized in that**
said scissor joints are joined to one another by a connecting rod mounted at the end of respectively one limb.

14. The ligament-tensioning device (1) according to one of the preceding claims,
**characterized in that**
said first and second proximal bearing plates (7, 9) each comprise an upper and a lower part (7a, 7b, 9a, 9b), wherein means for mounting the scissor-type guide (13) and optionally the spring element (15) are provided in the lower part (7a, 9a) and the upper part (7b, 9b) is fixed releasably on the lower part to increase, as desired, the thickness of the respective bearing plate.

15. The ligament-tensioning device (1) according to one of the preceding claims,
**characterized in that**
releasable locking means for independent locking of said first and second proximal bearing plates to said distal bearing plate at a minimum spacing and with respect thereto are provided on the distal bearing plate (5) or the first and second proximal bearing plate (7, 9).

16. The ligament-tensioning device (1) according to claim 15,
**characterized in that**
said locking means has respectively one hook pivotally mounted on the respective bearing plate and engaging in the opposing bearing plate, on which hook a first tool-engagement portion for actuation is provided.

17. The ligament-tensioning device (1) according to claim 16,
**characterized in that**
the first tool-engagement portion is formed for engagement of a hex key.

18. The ligament-tensioning device (1) according to claim 16 or 17,
**characterized in that**
from the material of said first and second proximal bearing plate (7, 9), in spatial association with the hook articulated on said distal bearing plate (5), there is formed an engagement portion for the hook.

19. The ligament-tensioning device (1) according to one of claims 16 to 18,
**characterized in that**
in each case a first bearing peg of a scissor joint of the first or second proximal bearing plate (7, 9) projects beyond the outer edge thereof and is so arranged relative to the hook pivotally mounted on said distal bearing plate (5) that it forms the counter-bearing of the locking means.

20. The ligament-tensioning device (1) according to one of claims 16 to 19,
**characterized in that**
in each case a second bearing peg of a scissor joint of said first or second proximal bearing plate (7, 9) projects beyond the outer edge thereof and is so arranged relative to the hook pivotally mounted on said distal bearing plate (5) that it forms a pivot axis thereof.

21. The ligament-tensioning device (1) according to one of the preceding claims,
**characterized in that**
on the side edges of the distal bearing plate (5) and of the first and second proximal bearing plate (7, 9) there is provided respectively one second tool-engagement portion, in particular a cylindrical recess, for engagement of a positioning tool.

22. The ligament-tensioning device (1) according to one of claims 3 to 21,
**characterized by**
a spring element (15) matched in spring constant to a predetermined tensioning force, which in particular provides a tensioning force that is substantially constant over its travel, in the range of between 50 and 90 N, in particular 70 N.

23. The ligament-tensioning device (1) according to one of the preceding claims,
**characterized in that**
the length/width ratio, i.e. the dimension in the medial-lateral direction with respect to that in the ventral-dorsal direction, is matched to the proportions of a resected portion of the adjacent skeletal part close to the joint.

24. The ligament-tensioning device (1) according to one of the preceding claims,
**characterized by**
a configuration matched to a knee joint, the adjacent skeletal parts being a femur and a tibia.

25. The ligament-tensioning device (1) according to claim 23 and 24,
**characterized in that**
the length/width ratio is 3/2 in accordance with that of a proximal resected tibia.

26. The ligament-tensioning device (1) according to one of the preceding claims,
**characterized by**
a configuration for releasable coupling of a sizer, which couples the proximal and distal skeletal parts in flexion in such a manner that axial transmission from the one skeletal part to the other is possible taking account of the ligament tension.

27. The ligament-tensioning device (1) according to claim 26,
**characterized by**
connecting means for fixing the sizer on the distal bearing plate (5).

28. The ligament-tensioning device (1) according to claim 27,
**characterized in that**
the connecting means comprise two clip-like extensions on the distal bearing plate which, each engaging around a side edge of the proximal bearing plates (7, 9), are guided to a base plate of the sizer.

29. Arrangement comprising a ligament-tensioning system (1) according to one of the preceding claims and a sizer which in particular are designed to match a knee joint and are releasably coupled together.

## Revendications

1. Dispositif tenseur de ligaments (1), destiné à activer le système ligamentaire et/ou capsulaire lors de l'implantation d'un implant articulaire, pourvu d'une plaque d'appui distale (5) destinée à être appuyée sur une partie distale du squelette et d'une première et deuxième plaques d'appui proximales (7, 9), recouvrant chaque fois partiellement la plaque d'appui distale (5) dans une première position d'utilisation, destinées à être appuyées sur une partie proximale du squelette et de moyens destinés au déplacement relatif des plaques d'appui proximales (7, 9), pour agrandir leur écart par rapport à la plaque d'appui distale,
l'ensemble des composants majeurs du dispositif tenseur de ligaments, notamment la plaque d'appui distale (5) et la première et deuxième plaques d'appui proximales (7, 9) étant dimensionnées pour être logées entre des tronçons proches de l'articulation de la partie distale et proximale du squelette, de telle sorte qu'après l'insertion du dispositif tenseur de ligaments, une capsule articulaire puisse à nouveau être fermée,
**caractérisé en ce que**
les moyens de déplacement comportent un guidage par cisaillement (13) supportant la plaque d'appui proximale concernée en position parallèle par rapport à la plaque d'appui distale, laquelle entre la plaque d'appui distale et la plaque d'appui proximale concernée est conçue de manière rigide en torsion, de sorte à mettre à disposition un guidage parallèle précis, dans la direction ventrale-dorsale, ainsi que médiale-latérale, entre la plaque d'appui distale et la plaque d'appui proximale.

2. Dispositif tenseur de ligaments (1) selon la revendication 1,
**caractérisé par**
des moyens exclusivement internes pour le déplacement relatif des plaques d'appui proximales par rapport à la plaque d'appui distale.

3. Dispositif tenseur de ligaments (1) selon l'une quelconque des revendications précédentes,
**caractérisé en ce que**
les moyens de déplacement comportent au moins un élément à ressort (15), destiné à entraîner les plaques d'appui proximales, lequel dans une position initiale, dans laquelle les plaques d'appui proximales (7, 9) présentent un écart minimal par rapport à la plaque d'appui distale (5) garde accumulée l'énergie d'entraînement et la libère lors de la mise en fonction du tendeur de ligaments, pour agrandir l'écart.

4. Dispositif tenseur de ligaments (1) selon la revendication 3,
**caractérisé en ce que**
les moyens de déplacement comportent, en association à la première et deuxième plaques d'appui proximales chaque fois un élément à ressort (15), destiné à entraîner les plaques d'appui proximales.

5. Dispositif tenseur de ligaments (1) selon la revendication 3 ou 4,
**caractérisé en ce que**
le ou chaque élément à ressort (15) est conçu de sorte à être verrouillable.

6. Dispositif tenseur de ligaments (1) selon l'une quelconque des revendications 3 à 5,
**caractérisé en ce que**
l'élément à ressort (15) comporte un élément à ressort de pression qui est placé dans une zone de recouvrement des plaques d'appui proximales et distale.

7. Dispositif tenseur de ligaments (1) selon la revendication 6,
**caractérisé en ce que**
l'élément à ressort (15) comporte une vis hélicoïdale en acier ou en titane, qui pour agrandir la course est réalisée sous forme de cône ou de double cône et comporte notamment une inclinaison indirectement proportionnelle au diamètre local des spires.

8. Dispositif tenseur de ligaments (1) selon la revendication 3 ou 4,
**caractérisé en ce que**
l'élément à ressort (15) comporte un élément à ressort de flexion.

9. Dispositif tenseur de ligaments (1) selon la revendication 8,
**caractérisé en ce que**
l'élément à ressort (15) comporte un ressort métallique en spirale ou un ressort à lames.

10. Dispositif tenseur de ligaments (1) selon l'une quelconque des revendications précédentes,
**caractérisé en ce que**
le guidage par cisaillement (13) comporte deux articulations à cisaillement placées à proximité d'extrémités en vis-à-vis de la plaque d'appui proximale.

11. Dispositif tenseur de ligaments (1) selon la revendication 10,
**caractérisé en ce que**
les extrémités des articulations à cisaillement sont insérées dans des rainures de la plaque d'appui distale ou proximale et chaque fois une branche ou chaque fois une extrémité des deux branches y est fixée en rotation par tourillons et tenons coulissants s'écoulant dans la direction d'extension de la rainure.

12. Dispositif tenseur de ligaments (1) selon la revendication 11,
**caractérisé en ce que**
sur les deux extrémités de la branche libre ou sur les extrémités respectivement libres des deux branches est prévu un tenon glissant, destiné à guider la branche ou l'extrémité en question dans une rainure de la plaque d'appui distale ou proximale, façonnée en adaptation au tenon glissant.

13. Dispositif tenseur de ligaments (1) selon l'une quelconque des revendications 10 à 12,
**caractérisé en ce que**
les articulations en cisaillement sont assemblées par l'intermédiaire d'une tige d'assemblage, montée sur l'extrémité de chaque fois une branche.

14. Dispositif tenseur de ligaments (1) selon l'une quelconque des revendications précédentes,
**caractérisé en ce que**
la première et la deuxième plaques d'appui proximales (7, 9) comprennent chacune une partie supérieure et inférieure (7a, 7b, 9a, 9b), dans la partie inférieure (7a, 9a) étant prévus des moyens de maintien du guidage par cisaillement (13) et en option, de l'élément à ressort (15) et **en ce que** pour l'agrandissement à volonté de l'épaisseur de la plaque d'appui en question, la partie supérieure (7b, 9b) est fixée de manière amovible sur la partie inférieure.

15. Dispositif tenseur de ligaments (1) selon l'une quelconque des revendications précédentes,
**caractérisé en ce que**
sur la plaque d'appui distale (5) ou la première et deuxième plaques d'appui proximales (7, 9) sont prévus des moyens de verrouillage amovibles, pour le verrouillage indépendant de la première et de la deuxième plaques d'appui proximales avec la plaque d'appui distale, avec un écart minimal et au vis-à-vis de celle-ci.

16. Dispositif tenseur de ligaments (1) selon la revendication 15,
**caractérisé en ce que**
les moyens de verrouillage comportent chacun un crochet logé en pivotement sur la plaque d'appui en question et s'engageant dans la plaque d'appui opposée, sur lequel est prévu un premier segment d'engagement d'outil pour l'actionnement.

17. Dispositif tenseur de ligaments (1) selon la revendication 16,
**caractérisé en ce que**
le premier segment d'engagement d'outil est façonné pour l'engagement d'une clé Allen standard.

18. Dispositif tenseur de ligaments (1) selon la revendication 16 ou 17,
**caractérisé en ce**
**qu'**à partir de la matière de la première et de la deuxième plaques d'appui proximales (7, 9), en association physique au crochet articulé sur la plaque d'appui distale (5) est façonné un segment d'engagement pour celui-ci.

19. Dispositif tenseur de ligaments (1) selon l'une quelconque des revendications 16 à 18,
**caractérisé en ce que**
chaque fois un premier tenon d'une articulation par cisaillement de la première et de la deuxième plaques d'appui proximales (7, 9) déborde par-dessus leur bord extérieur et est placé par rapport au crochet pivotant sur la plaque d'appui distale (5), de sorte à former la butée des moyens de verrouillage.

20. Dispositif tenseur de ligaments (1) selon l'une quelconque des revendications 16 à 19,
**caractérisé en ce que**
chaque fois un deuxième tenon d'une articulation par cisaillement de la première et de la deuxième plaques d'appui proximales (7, 9) déborde par-dessus leur bord extérieur et est placé par rapport au crochet pivotant sur la plaque d'appui distale (5), de sorte à former son axe de pivotement.

21. Dispositif tenseur de ligaments (1) selon l'une quelconque des revendications précédentes,
**caractérisé en ce que**
sur les arêtes latérales de la plaque d'appui distale (5) et de la première et de la deuxième plaques d'appui proximales (7, 9) est prévu chaque fois un tronçon d'engagement d'outil, notamment un évidement cylindrique, pour l'engagement d'un outil de positionnement.

22. Dispositif tenseur de ligaments (1) selon l'une quelconque des revendications 3 à 21,
**caractérisé par**
un élément à ressort (15) dont la rigidité de ressort est adaptée à une force de tension prédéfinie, lequel met à disposition notamment une force de tension sensiblement constante sur sa course, de l'ordre compris entre 50 et 90 N, notamment de 70 N.

23. Dispositif tenseur de ligaments (1) selon l'une quelconque des revendications précédentes,
**caractérisé en ce que**
le rapport longueur/largeur, donc la dimension dans la direction médiale-latérale, par rapport à celle dans la direction ventrale-dorsale est adaptée aux proportions d'un tronçon coupé, proche de l'articulation de la partie de squelette voisine.

24. Dispositif tenseur de ligaments (1) selon l'une quelconque des revendications précédentes,
**caractérisé par**
la réalisation en adaptation à une rotule, les parties de squelette voisines étant un fémur et un tibia.

25. Dispositif tenseur de ligaments (1) selon la revendication 23 et 24,
**caractérisé en ce**
**qu'**en correspondance à un tibia proximal coupé, le rapport longueur/largeur correspond à 3/2.

26. Dispositif tenseur de ligaments (1) selon l'une quelconque des revendications précédentes,
**caractérisé par**
une conception pour un accouplement amovible d'un calibreur, lequel accouple la partie proximale et distale du squelette en flexion, de telle sorte qu'une transmission d'axe de l'une des parties du squelette sur l'autre soit possible sous considération de la tension des ligaments.

27. Dispositif tenseur de ligaments (1) selon la revendication 26,
**caractérisé par**
des moyens d'assemblage, destinés à fixer le calibreur sur la plaque d'appui distale (5).

28. Dispositif tenseur de ligaments (1) selon la revendication 27,
**caractérisé en ce que**
les moyens d'assemblage comportent deux prolongements en forme de crampons sur la plaque d'appui distale, lesquels, en enserrant chacun une arête latérale des plaques d'appui proximales (7, 9) sont guidés vers la plaque d'embase du calibreur.

29. Agencement d'un dispositif tenseur de ligaments (1) selon l'une quelconque des revendications précédentes et d'un calibreur, qui sont réalisés et accouplés l'un à l'autre de manière amovible, notamment en adaptation à une rotule.
